(19) Europäisches Patentamt
European Patent Office
Office européen des brevets

(11) Publication number: 0 080 276 A1

(12) # EUROPEAN PATENT APPLICATION

(21) Application number: 82305792.2

(22) Date of filing: 01.11.82

(51) Int. Cl.³: C 07 C 107/02, C 08 F 4/04

(30) Priority: 19.11.81 GB 8134921

(43) Date of publication of application: 01.06.83 Bulletin 83/22

(84) Designated Contracting States: BE DE FR GB IT NL

(71) Applicant: IMPERIAL CHEMICAL INDUSTRIES PLC, Imperial Chemical House Millbank, London SW1P 3JF (GB)

(72) Inventor: Davies, Stephen Parry, 26 Clara Street South Yarra, Melbourne Victoria 3141 (AU)
Inventor: Thompson, Morice William, "Urishay" Highfield Lane, Cox Green Maidenhead, Berkshire (GB)

(74) Representative: Wood, Dennis John Cecil et al, Imperial Chemical Industries PLC Legal Department: Patents Thames House North Millbank, London SW1P 4QG (GB)

(54) **Azocarboxylic esters useful as polymerisation initiators.**

(57) Azocarboxylic esters containing in the molecule poly(alkyleneoxy) residues have physical properties which render them of especial interest and utility as free radical-type polymerisation initiators.

EP 0 080 276 A1

0080276
Pv. 32101/EP.

# AZOCARBOXYLIC ESTERS USEFUL AS POLYMERISATION INITIATORS

This invention relates to novel organic azo compounds which are useful as initiators for the polymerisation of ethylenically unsaturated monomers.

The use of organic azo compounds as initiators in the polymerisation of ethylenically unsaturated monomers is well known and extensively described in the literature. Illustrative of such compounds are 2,2'-azobis(2-methylpropionitrile), 4,4'-azobis(4-cyanopentanoic acid), 2,2'-azobis(2-cyanoethyl-p-benzoic acid), 4,4'-azobis-(4-cyano-n-pentanol), 2,2'-azobis(4-amino-2, 4-dimethylvaleronitrile) and 1,1'-azobis(1-cyanocyclohexane).

According to the present invention there are provided azo carboxylic esters having the general formula

$$R_1 - \underset{R_3}{\overset{R_2}{\overset{|}{\underset{|}{C}}}} - N = N - \underset{R_5}{\overset{R_4}{\overset{|}{\underset{|}{C}}}} - COO(C_nH_{2n}O)_p - R \qquad (I)$$

wherein R is the residue of an aliphatic monohydroxy compound after the removal of the hydroxyl group therefrom, $R_1$ is a methyl or ethyl group or a grouping of the formula $-COO(C_nH_{2n}O)_p - R$; $R_2$ is a methyl group or a β-carboxyethyl group and $R_3$ is a methyl group or an ethyl group, or $R_2$ and $R_3$ together with the intermediate carbon atom form a cyclohexyl group; $R_4$ is a methyl group and $R_5$ is a methyl group, an ethyl group, a β-carboxyethyl group or a β-methoxy-ββ-dimethylethyl group, or $R_4$ and $R_5$ together with the intermediate carbon atom form a cyclohexyl group; n has the value 2, 3 or 4 and p is an integer from 2 to 100,

more particularly from 2 to 50.

The ester moiety of the compounds (I) is thus derived from a poly(alkyleneoxy) condensate of the hydroxy compound R-OH. The poly(alkyleneoxy) chain may consist entirely of ethyleneoxy units, or of propyleneoxy units or of butyleneoxy units, or it may include units of any two or all three of these types. Furthermore, in the latter case, the different units may be arranged randomly within the chain or they may occur in blocks. Examples of such hydroxy compounds include the monomethyl ether of polyethylene glycol of molecular weight 250, in which R is $CH_3$, n is 2 and p is approximately 5; the monomethyl ether of polyethylene glycol of molecular weight 2000, in which R is $CH_3$, n is 2 and p is approximately 45; the random condensate of ethylene oxide and propylene oxide with methanol in the approximate molar proportions 24:6:1, with a molecular weight of 1450-1750, in which R is $CH_3$, n is 2 and 3 and p is approximately 30; and the monomethyl ether of diethylene glycol, in which R is $CH_3$, n is 2 and p is 2.

The esters of the invention may be obtained from the corresponding azonitrile by a two-step process; in the first step, the nitrile is subjected to the Pinner reaction with the appropriate compound R-OH in the presence of hydrogen chloride gas under anhydrous conditions to form the intermediate iminoether hydrochloride, and in the second step the iminoether hydrochloride is hydrolysed to yield the required carboxylic ester. According to the known procedure, the Pinner reaction of a nitrile is conducted using a stoichiometric excess of the alcohol and, in suitable cases, the iminoether hydrochloride then precipitates out and is separated from the reaction mixture. However, the intermediate isolation of the iminoether salt

may prove difficult where the alcohol is, as in the present case, a poly(alkyleneoxy) compound. We have found that, in these specific circumstances, the amount used of the compound in question may be limited to that which is stoichiometrically equivalent to the nitrile groups present in the azonitrile. The conversion of the azonitrile to the iminoether is substantially quantitative and it is possible to proceed directly to the hydrolysis step without isolation of its hydrochloride. This procedure is the subject of, and is more fully described in, our co-pending Application No. 8134919.

Azonitriles from which the azoesters of the invention may be derived include the following which are commercially available : 2,2'-azobis(2-methylpropionitrile), 2,2'-azobis(2-methylbutyronitrile), 4,4'-azobis(4-cyanopentanoic acid), 1,1'-azobis(1-cyanocyclohexane), 2-(2'-methylpropan-2'-azo)-2-cyano-4-methoxy-4-methylpentane, 1-(2'-methylpropan-2'-azo)-1-cyanocyclohexane, 1-(2'-methylbutan-2'-azo)-1-cyanocyclohexane, 2-(2'-methylpropan-2'-azo)-2-cyanopropane and 2-(2'-methylpropan-2'-azo)-2-cyanobutane.

Azo carboxylic esters according to the invention are valuable as free radical-forming initiators for the polymerisation of ethylenically unsaturated monomers. According to their physical properties, they may be especially suitable for specific polymerisation procedures. Thus, certain members of the class are preferentially soluble in organic media and are liquid at ordinary temperatures, and are consequently very convenient for initiating polymerisation of monomers in bulk or in solution; their liquid nature much facilitates their measuring and dispensing into the polymerisation mixture. An example of

such a compound is that already mentioned which is derived from 2,2'-azobis(2-methylpropionitrile) and in which the ester moiety in formula (I) is derived from a random condensate of ethylene oxide and propylene oxide with methanol in the molar ratio 24:6:1. Certain other members of the class are, in contrast, preferentially soluble in water and are therefore useful in polymerisations carried out by aqueous emulsion techniques. An example of such a compound is that already mentioned, again derived from 2,2'-azobis(2-methylpropionitrile), in which the ester moiety in formula (I) is derived from the monomethyl ether of polyethylene glycol of molecular weight 2000.

The invention is illustrated but not limited by the following Examples.

## EXAMPLE 1

The monomethyl ether of polyethylene glycol of molecular weight 1950 (238 g, 1 molar equivalent) was dried by azeotropic distillation with toluene and then dissolved in dichloromethane (300 ml.).2,2'-Azobis(2-methylpropionitrile) (10.01 g, 0.5 molar equivalent) was added and dry hydrogen chloride gas was passed through the solution with cooling and stirring, keeping the temperature below about 10°C. After about 8 hours, the solution had absorbed about 30 g of hydrogen chloride and was saturated. The reaction mixture was then allowed to stand at room temperature for 168 hours. Infra-red examination of the mixture at the end of this period indicated almost complete absence of the characteristic -C≡N band at 2250 $cm^{-1}$. After the reaction mixture had stood at room temperature for a total of 264 hours, the solvents were removed under reduced pressure. The residue was poured into cold water (about 200 ml.) and extracted with dichloromethane (about 200 ml.).

The organic phase was separated, dried with anhydrous magnesium sulphate and then concentrated to dryness under reduced pressure. The product was a white, waxy solid which was soluble in water; it was also soluble in methyl methacrylate but, in the presence of both water and methyl methacrylate, it was preferentially soluble in the water phase. When examined by infra-red spectroscopy it exhibited a characteristic ester grouping peak at 1735 $cm^{-1}$; there was no peak observable at 2250 $cm^{-1}$, indicating the absence of nitrile groups. Thin-layer chromatography of the product and of the parent monomethylether of polyethylene glycol demonstrated that the two compounds were readily separated and distinguished; there was no evidence of contamination of the product with this starting material. It was concluded therefore that the product was substantially the ester of polyethylene glycol(1950)monomethylether and 2,2'-azobis(2-methylpropanoic acid), as represented by the following formula:-

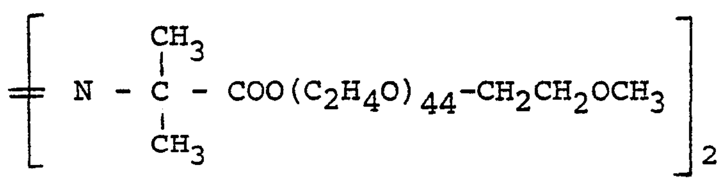

## EXAMPLE 2

The condensate of methanol (1 mol) with a mixture of ethylene oxide (24 mols) and propylene oxide (6 mols) (123g, 0.07 mol.) was dried by azeotropic distillation with toluene and then dissolved in dichloromethane (300g.), 2,2'-Azobis(2-methylpropionitrile)(5.74g, 0.035 mol) was added and dry hydrogen chloride gas was bubbled into the cooled and stirred reaction mixture, keeping the temperature below about 10°C. After about 15g of hydrogen chloride had been passed, the solution was saturated. The mixture was then

allowed to stand at room temperature for 5 days, following which the solvent and hydrogen chloride were removed under reduced pressure. The residue was stirred with water (200 ml) and extracted with dichloromethane. Removal of the solvent from the extract under reduced pressure gave a colourless liquid which was insoluble in water. Examination of the product by infra-red spectroscopy indicated complete absence of nitrile groups; the product was concluded to be substantially the ester of the methanol/ethylene oxide/propylene oxide condensate and 2,2'-azobis(2-methylpropanoic acid), as represented by the formula:-

$$\left[ = N - \underset{CH_3}{\overset{CH_3}{\overset{|}{\underset{|}{C}}}} - COO(C_2H_4O)_{24}\ (C_3H_6O)_6\ OCH_3 \right]_2$$

EXAMPLE 3

The monomethyl ether of polyethylene glycol of molecular weight 250 (50g, 0.2 mol) was dried by azeotropic distillation with toluene and dissolved in dichloromethane (100 ml).2,2'-Azobis(2-methylpropionitrile) (16.4g, 0.1 mol) was added and dry hydrogen chloride gas was passed into the stirred and cooled solution until it was saturated, keeping the temperature below about 10°C. After the reaction mixture had stood at room temperature for 7 days, the solvent and hydrogen chloride were removed under reduced pressure. The residue was added to water (100 ml) and extracted with dichloromethane (150 ml). The extract was dried over anhydrous magnesium sulphate and then concentrated under reduced pressure to give a yellow liquid which was soluble in methyl methacrylate but only very slightly soluble in water. It was an efficient emulsifier for methyl methacrylate/water mixtures. Examination of the product by infra-red spectroscopy revealed a strong characteristic ester group absorption and absence of any nitrile group absorption. When subjected to thin-layer chromatography, the product exhibited a single streak which migrated differently from the monomethyl ether of the polyethylene glycol. The product was concluded to be substantially the ester of polyethylene glycol(250)monomethyl ether and 2,2'-azobis-(2-methylpropanoic acid) as represented by the formula:-

$$\left[ = N - \underset{CH_3}{\overset{CH_3}{\underset{|}{\overset{|}{C}}}} - COO(C_2H_4O)_5\ CH_2CH_2OCH_3 \right]_2$$

EXAMPLE 4

Dry diethylene glycol monomethyl ether (52.2g, 0.44 mol.) and 2,2' azobis(2-methylpropionitrile)(36.1g,0.22 mol) were together dissolved in dichloromethane (100 ml). Dry hydrogen chloride gas was bubbled through the cooled and stirred solution until it was saturated (about 15g of hydrogen chloride had been passed at this stage), and keeping the temperature below about 10°C. The reaction mixture was allowed to stand at room temperature for 7 days and the solvent and hydrogen chloride were then removed under reduced pressure. The residue was poured into cold 10% aqueous potassium hydrogen carbonate (150 ml) and stirred. After 10 minutes, the mixture was extracted with dichloromethane (2 portions of 100 ml) and the extract concentrated under reduced pressure. The product was a yellow liquid which subsequently solidified to form needle-shaped colourless crystals. Infra-red spectroscopic examination of the product showed a strong ester group absorption and an absence of nitrile group absorption. The product was very slightly soluble in water, but soluble in methyl methacrylate. It was concluded to be substantially 2,2'-azobis-[(3,6-oxaheptyl)2-methylpropanoate], as represented by the formula:-

$$\left[ = N - \overset{\displaystyle CH_3}{\underset{\displaystyle CH_3}{\overset{|}{\underset{|}{C}}}} - COO.CH_2CH_2O\ CH_2CH_2\ O\ CH_3 \right]_2$$

WHAT WE CLAIM IS:-

1. Azocarboxylic esters having the general formula

$$R_1 - \underset{R_3}{\overset{R_2}{C}} - N = N - \underset{R_5}{\overset{R_4}{C}} - COO(C_nH_{2n}O)_p - R \qquad (I)$$

wherein R is the residue of an aliphatic monohydroxy compound after the removal of the hydroxyl group therefrom, $R_1$ is a methyl or ethyl group or a grouping of the formula $-COO(C_nH_{2n}O)_p - R$; $R_2$ is a methyl group or a β-carboxyethyl group and $R_3$ is a methyl group or an ethyl group, or $R_2$ and $R_3$ together with the intermediate carbon atom form a cyclohexyl group; $R_4$ is a methyl group and $R_5$ is a methyl group, an ethyl group, a β-carboxyethyl group or a β-methoxy-ββ-dimethylethyl group, or $R_4$ and $R_5$ together with the intermediate carbon atom form a cyclohexyl group; n has the value 2, 3 or 4 and p is an integer from 2 to 100.

2. Esters as claimed in claim 1, wherein p is an integer from 2 to 50.

3. Esters as claimed in claim 1 or claim 2, wherein $R_1$ is a grouping of the formula $-COO(C_nH_{2n}O)_p - R$ and $R_2$, $R_3$, $R_4$ and $R_5$ are all methyl groups.

4. Esters as claimed in claim 3, wherein R is a methyl group, n is 2 and p is an integer from 5 to 45.

5. Esters as claimed in claim 3, wherein R is a methyl group, n is 2 and 3 and p is approximately 30.

6. Azocarboxylic esters according to claim 1 substantially as hereinbefore described with reference to the foregoing Examples.

0080276

European Patent Office

# EUROPEAN SEARCH REPORT

Application number

EP 82 30 5792

## DOCUMENTS CONSIDERED TO BE RELEVANT

| Category | Citation of document with indication, where appropriate, of relevant passages | Relevant to claim | CLASSIFICATION OF THE APPLICATION (Int. Cl. 3) |
|---|---|---|---|
| X | CHEMICAL ABSTRACTS, vol. 86, no. 10, 7th March 1977, page 24, no. 56004x, Columbus Ohio (USA); D.E.GREGONIS et al.: "The chemistry fo some selected methacrylate hydrogels". & ACS SYMP. SER. 1976, 31(HYDROGELS MED. RELAT. APPL., SYMP., 1975), 88-104. *Abstract* | 1-3,6 | C 07 C 107/02<br>C 08 F 4/04 |
| X | CHEMICAL ABSTRACTS, vol. 87, no. 2, 10th July 1977, page 25, no. 6707d, Columbus Ohio (USA); D.E.GREGONIS et al.: "The chemistry of methacrylate hydrogels". & POLYM. PREPR., AM. CHEM. SOC., DIV. POLYM. CHEM. 1975, 16(2), 349-53. *Abstract* | 1-3,6 | TECHNICAL FIELDS SEARCHED (Int. Cl. 3) |
| X | DE-A-2 124 000 (MERCK PATENT GmbH) *Claims; examples 6-8,10,11* | 1 | C 07 C 107/00 |

The present search report has been drawn up for all claims

| Place of search | Date of completion of the search | Examiner |
|---|---|---|
| THE HAGUE | 14-02-1983 | PAUWELS G.R.A. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document